# EUROPEAN PATENT APPLICATION

(11) **EP 2 047 826 A1**
(43) Date of publication of application: **15.04.2009**
(21) Application number: 08165803.1
(22) Date of filing: 03.10.2008
(51) Int. Cl.: A61F 2/46

(54) **Modular stem inserter**

(30) Priority: 10.10.2007 US 869762
(71) Applicant: DePuy Products, Inc., Warsaw, IN 46581 (US)
(72) Inventor: Maness, Megan A., Warsaw, IN 46580 (US); Furbee, Rebecca M., Warsaw, IN 46582 (US); Alford, Stephen A., Alexandria, IN 46001 (US)
(74) Representative: Belcher, Simon James

(57) **Abstract**

A kit for use in implanting a stem into a long bone includes a universal handle (12) including a locking mechanism (30), and a plurality of shafts (14). Each of the plurality of shafts is adapted to couple with the universal handle such that the locking mechanism of the universal handle locks each of the plurality of shafts to the handle.

## Description

The present invention relates generally to the field of orthopaedics, and more particularly, to an instrument used to insert an implant for use in arthroplasty.

Patients who suffer from the pain and immobility caused by osteoarthritis and rheumatoid arthritis have an option of joint replacement surgery. Joint replacement surgery is quite common and enables many individuals to function properly when it would not be otherwise possible to do so. Artificial joints are usually comprised of metal, ceramic and/or plastic components that are fixed to existing bone.

Such j oint replacement surgery is otherwise known as j oint arthroplasty. Joint arthroplasty is a well-known surgical procedure by which a diseased and/or damaged joint is replaced with a prosthetic joint. In a typical total joint arthroplasty, the ends or distal portions of the bones adjacent to the joint are resected or a portion of the distal part of the bone is removed and the artificial joint is secured thereto.

There are known to exist many designs and methods for manufacturing implantable articles, such as bone prostheses. Such bone prostheses include components of artificial joints such as elbows, hips, knees and shoulders.

Numerous instruments are required in performing a hip arthroplasty. The surgeon must use various reamers and broaches for cutting and shaping the bone. Additionally, when implanting the stem into the long bone, the surgeon must use a stem inserter.

In hip arthroplasty, there are currently many different approaches, or surgical techniques in implanting the bone prostheses. The posterior approach accesses the joint through the back, gives straightforward access to the acetabulum, provides good visualization of the femoral shaft, and allows the surgeon to preserve the hip abductors. The anterolateral approach exploits the intermuscular plane between the tensor fasciae latae and the gluteus medius, involves partial or complete detachment of the abductor mechanism, and combines good exposure of the acetabulum with safety during preparation of the femoral shaft. The anterior approach utilizes the internervous plane between the sartorius and the tensor fasciae latae, exposes the hip without detachment of muscle from the bone, and takes advantage of the fact that the hip is an anterior joint, closer to the skin anteriorly than posteriorly.

Because femoral access is different with each of the approaches mentioned above, it is desirable to use different stem inserters for each approach. Depending on how the surgeon approaches the femur, or long bone, an inserter with different angular or curved configurations may be preferred so as to best reach the stem/implant without impinging the bone or surrounding soft tissue. Surgeons may also choose different angled/curved/ offset inserters depending on the anatomy of the individual patients and the selected implants. Some surgeons also prefer to use multiple stem inserters at the varying stages of stem insertion during a single surgery. Like other surgical instruments, these stem inserters are housed in instrument cases that must conform to weight and size requirements. Unfortunately, the handles of the stem inserters are quite bulky and can greatly reduce the available case weight and free space for other required hip arthroplasty instrumentation. The transportation, set-up, and sterilization of multiple heavy stem inserters can also become a burden to the surgical staff. Therefore, there is a need for a reduction in the size and weight of surgical instruments used in joint replacement surgeries.

In one aspect, the invention provides a kit for use in implanting a stem into a long bone, which includes a universal handle including a locking mechanism and a plurality of shafts. Each of the plurality of shafts is adapted to couple with the universal handle such that the locking mechanism of the universal handle locks each of the plurality of shafts to the handle.

In another aspect, the invention provides a kit for use in implanting a stem in a long bone, which includes a handle and a removable shaft. The removable shaft includes an end portion for extending into the aperture to lock the removable shaft to the handle.

The kit of the invention can be used in a method for inserting a stem into a long bone, which includes providing a handle and a plurality of shafts, each of the plurality of shafts including a handle-attachment end and a stem-attachment end. One of the plurality of shafts is selected and the handle-attachment end of the selected shaft is inserted into the handle. The stem-attachment end of the shaft is inserted into the stem and the stem is seated into the long bone.

Embodiments of the invention are described below by way of example with reference to the accompanying drawings, in which:
FIG. 1 is a plan view of a modular stem inserter according to one embodiment of the present invention;
FIG. 2 is a top view of the modular stem inserter of FIG. 1;
FIG. 3 is a side view of the modular stem inserter of FIG. 1;
FIG. 4 is an internal, assembled view of a locking mechanism of the handle of the stem inserter according to one embodiment of the present invention;
FIG. 5 is an exploded view of the locking mechanism of the stem inserter handle of FIG. 4 and the stem inserter shaft of FIG. 1.
FIG. 6 is an internal, assembled view of the stem inserter handle of FIG. 4 and stem inserter shaft of FIG. 1
FIG. 7 is a plan view of a modular stem inserter kit according to one embodiment of the present invention.

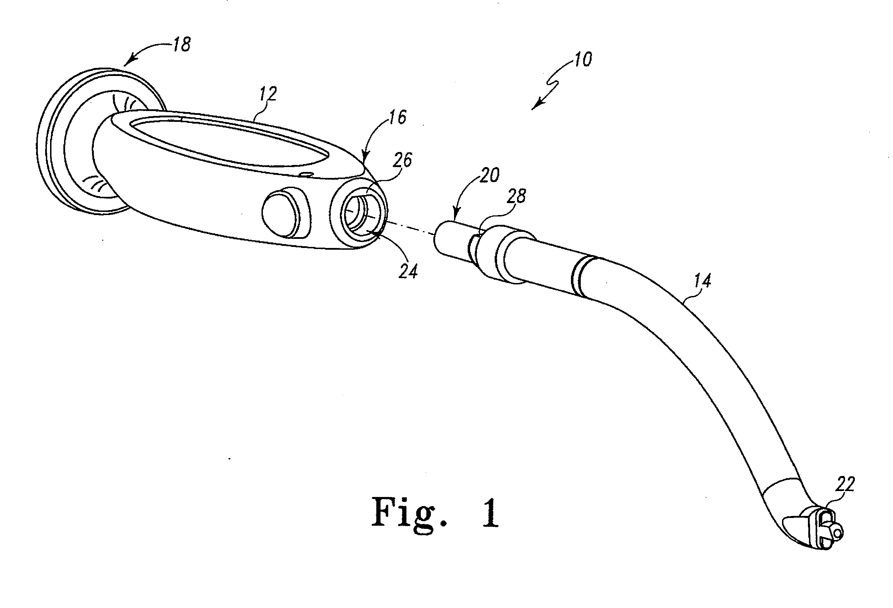

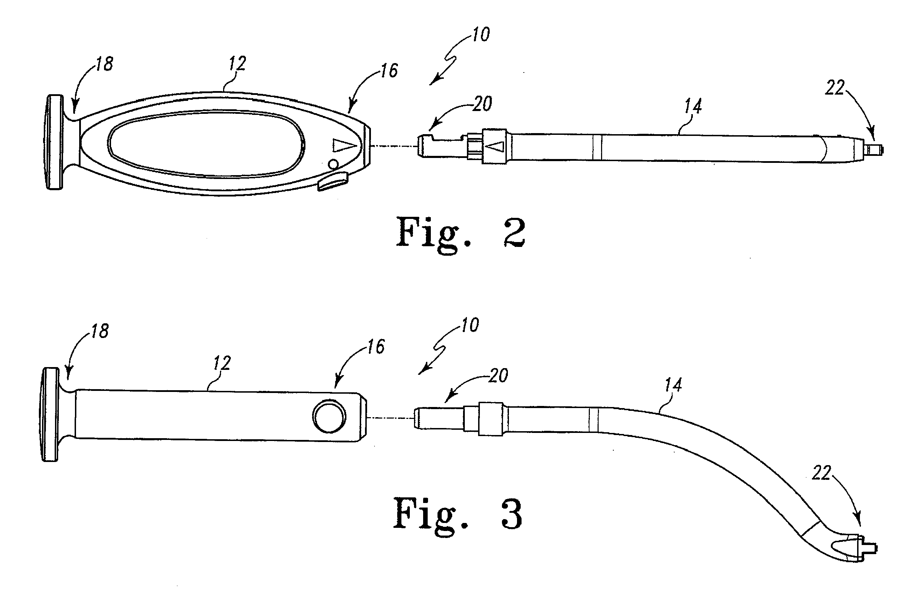

Referring to the drawings, FIGS. 1 to 3 show a stem inserter 10 which includes a handle 12 and a shaft 14. In the embodiment illustrated in FIG. 1, the shaft 14 is a curved anterior shaft used during the anterior approach. However, it should be understood that any type of shaft may be used, and the curved anterior shaft is for illustrative purposes only.

As shown in FIG. 1, the handle 12 is separable from the shaft 14. The handle 12 includes a shaft-attachment end 16 and an opposing end 18. The shaft 14 includes a handle-attachment end 20 and a stem attachment end 22. The stem attachment end 22 is designed to fit on to the end of a stem (not shown) for insertion into the long bone. The stem attachment end 22 of the shaft 14 may vary depending on the stem's insertion feature. The shaft-attachment end 16 of the handle 12 includes an aperture 24 designed to receive the handle-attachment end 20 of the shaft 14. In the illustrated embodiment, the aperture 24 includes a flat end 26 that corresponds to a flat end 28 on the handle-attachment end 20. The flat ends 26, 28 ensure proper alignment of the shaft 14 and the handle 12 while also minimizing rotational toggle. The opposing end 18 of the handle 12 serves as an impaction surface for the surgeon during stem insertion.

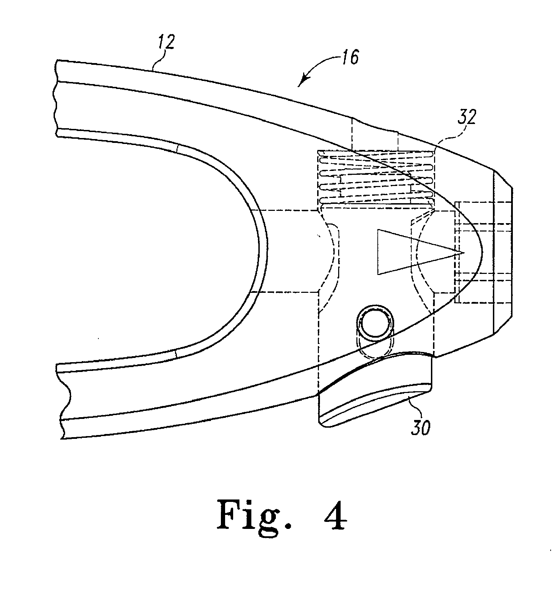

FIG. 4 shows an internal, assembled view of the shaft-attachment end 16 of the handle 12. The shaft-attachment end 16 of the handle 12 includes a locking mechanism for securing the handle 12 to the shaft 14. The locking mechanism includes a spring-loaded button 30. The spring-loaded button 30 includes a spring 32 to lock the shaft 14 to the handle 12.

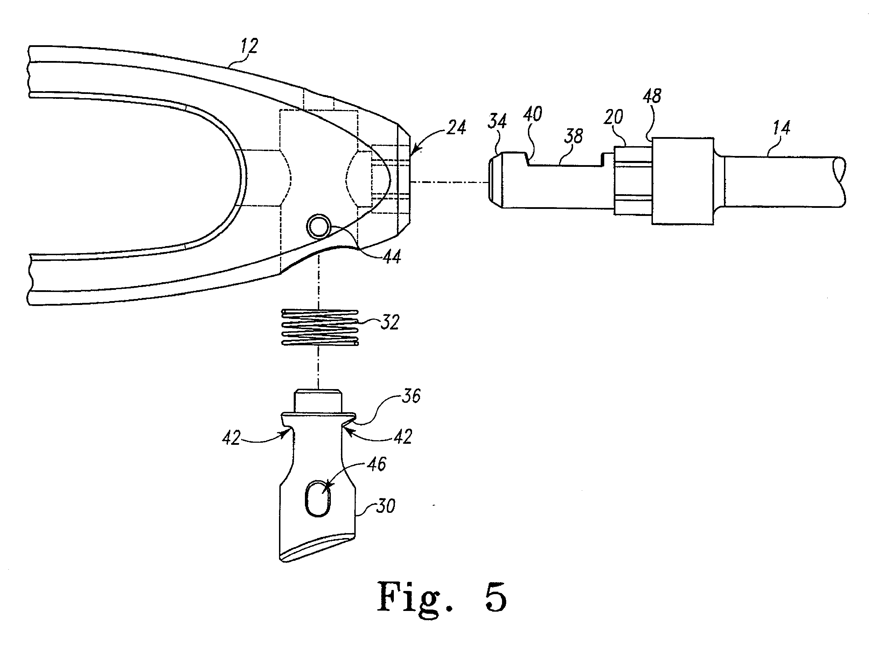

FIG. 5 shows an exploded view of the handle 12 and the shaft 14. The handle-attachment end 20 of the shaft 14 includes a shaft tip chamfer 34. The angle on the chamfer 34 allows the shaft 14 to be inserted into the aperture 24 of the handle 12 without depressing the button 30. The chamfer 34 presses against a corresponding edge 36 of the button 30, which causes the button 30 to compress the spring 32, thus allowing the shaft 14 to be inserted.

The shaft 14 also includes a shaft-locking slot 38 and a shaft-locking ramp 40. When the shaft 14 is inserted into the handle 12, the shaft-locking slot 38 and the shaft-locking ramp 40 engage with the internal button base 42 of the button 30 in order to lock the shaft 14 into the handle 12. Specifically, once the shaft 14 is inserted into the handle 12, the spring 32 decompresses causing the button 30 to apply force to the shaft-locking ramp 40. This motion forces the shaft 14 up and into the handle 12 to minimize toggle and ensure a secure lock. Once the shaft 14 is inserted into the handle 12, the shaft-locking slot 38 receives pressure from the button 30 to lock the shaft 14 to the handle 12. The shaft-locking slot 38 also prevents the shaft 14 from being pulled out of the handle 12 without first depressing the button 30.

A stop pin 44 is included on the handle 12 and engages a slot 46 on the button 30. The stop pin 44 prevents the button 30 from falling out of the handle 12. Also, the slot 46, along with the stop pin 44, limits the travel of the button 30 in order to ensure that the locking mechanism of the handle 12 always functions properly.

Also as shown in FIG. 5, the shaft 14 includes a flat impaction surface 48. The flat impaction surface 48 allows the surgeon's impaction force to transfer from the handle 12 to the shaft 14. The flat-impaction surface 48 ensures that excessive load is not applied to the various features of the locking mechanism (e.g., the spring-loaded button 30, the shaft-locking slot 38, etc...).

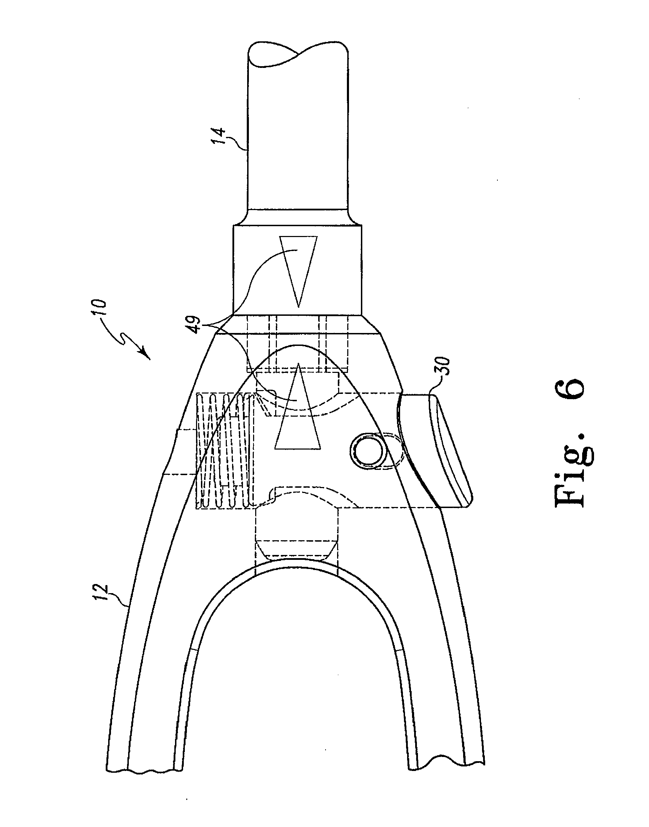

FIG. 6 shows the modular stem inserter 10 with the handle 12 and the shaft 14 in an engaged position. As shown, the spring-loaded button 30 has engaged the shaft-locking slot 38 (FIG. 5) of the shaft 14. The spring 32 (FIG. 5) is in a compressed position pushing the button 30 out, causing the internal button base 42 (FIG. 5) of the button 30 to contact shaft-locking ramp 40 (FIG. 5), locking the shaft 14 to the handle 12. As identified in FIG. 6, the handle 12 and the shaft 14 have each been etched with an alignment triangle 49. The alignment triangles 49 indicate alignment of the flat end 26 (FIG. 1) of the handle 12 and the flat end 28 (FIG. 1) of the shaft 14, thus acting as a visual for easy insertion. This feature allows the user to insert the handle-attachment end 20 of the shaft 14 into the aperture 24 of the handle 12 in any rotational orientation and then simply rotate the shaft 14 until the two alignment triangles 49 are aligned. The shaft 14 then snaps into the handle 12.

Although in the embodiment shown in the drawings, the alignment features 49 are triangular, other shapes or indicators could be used. Also, other known marking methods instead of etching may also be used to create these features. In some embodiments, the etchings 49 may not be included and instead, the user may assemble the handle 12 and the shaft 14 by feel.

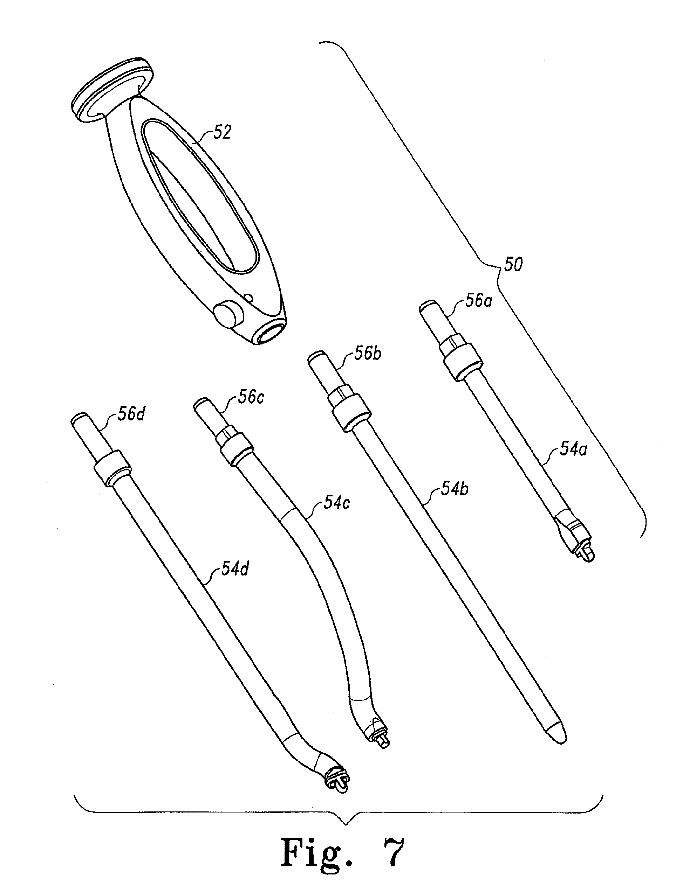

Turning now to FIG. 7, a kit 50 according to one embodiment of the present invention is shown. In this embodiment, the kit 50 includes a handle 52 and four shafts 54a, 54b, 54c, 54d. Each of the shafts 54a, 54b, 54c, 54d includes a handle-attachment end 56a, 56b, 56c, 56d that is configured the same as the handle-attachment end 20 illustrated in FIGS. 1 to 6 above. In these embodiments, because each of the handle-attachment ends 56a, 56b, 56c, 56d is of the same design, each shaft can be locked into the handle depending on the type of surgery or the surgeon preference. The illustrated kit includes the standard straight shaft 54a, the curved anterior shaft 54b, the posterior shaft 54c, and the bullet tip shaft 54d.

During surgery, the surgeon will select the appropriate shaft based upon the surgical approach, the surgeon's personal preference, and patient anatomy, as well as the type of stem being implanted. The surgeon will attach the selected shaft 54 to the handle 52 by pushing the two together, making sure the two alignment arrows 49 (FIG. 6) of the handle 12 and the shaft 14 are aligned. After introducing the stem by hand into the femoral canal, the surgeon then aligns the stem attachment end 22 (FIGS. 1 to 6) of shaft 14 with the stem's driver platform and continues the insertion process until the stem is properly seated. In some embodiments, the stem-attachment end 22 of the shaft 14 may be threaded to correspond to threads in the stem. In such a case, the surgeon could then thread the stem-attachment end 22 of the shaft 14 on to the stem and use the stem inserter 10 to introduce the stem into the femoral canal.

In one embodiment, the handle 12 and the shaft 14 will be manufactured of a metal material. This metal could be a stainless steel including, but not limited to, precipitation hardening stainless steels such as 17-4, 13-8Mo, XM-13, 455, XM-25, and 465 or martensitic stainless steels such as 410, 416, 420, 431, 440A, 440B, and 440C. The instruments could also be manufactured out of a cobalt-based alloy such as wrought CoCrMo (F1537), a hardened condition of Co-Cr-W-Ni (F90), cold worked MP35N (ASTM F562), or another metal material suitable for a medical application. In other embodiments, the handle 12 may be made of re-usable stainless steel while the shaft 14 is a disposable device made of a plastic material. This plastic may or may not contain reinforcement and could be ABS, polypropylene, polyurethane, polyesters, acetals, or polyimide. This is a representative list and does not exclude other plastics or polymer systems that are used for medical applications.

Although the embodiments discussed above depict four different types of shafts, any number of shafts may be included. The shafts may be shaped to relate to a particular surgical technique, a different type of stem, a surgeon's preference, or even a particular stage of the impaction process. The shafts may also have a threaded stem attachment end 22 in order to provide the surgeon greater version and insertion control.

Although the locking mechanism has been described as a spring-loaded button cooperating with a shaft-locking slot, other types of known locking mechanisms may be utilized such as a ball plunger, interlocking teeth, Hudson end, prongs and/or circular springs.

In other embodiments, the shaft-tip chamfer may not be utilized, and the user may have to activate the locking mechanism by depressing the button 30 in order to insert the shaft into the handle.

## Claims

1. A kit for use in implanting a stem in a long bone, the kit including:
a handle;
a removable shaft, the removable shaft including an end portion for extending into the aperture to lock the removable shaft to the handle.

2. The kit of claim 1, in which the handle is made of a metal selected from the group of stainless steels.

3. The kit of claim 1, in which the handle includes a locking mechanism for locking the handle to the removable shaft.

4. The kit of claim 3, in which the locking mechanism includes a spring-loaded button.

5. The kit of claim 4, in which the removable shaft comprises shaft-tip chamfer for allowing the removable shaft to be inserted into the handle without activating the spring-loaded button.

6. The kit of claim 4, in which the removable shaft includes a shaft-locking slot and a shaft-locking ramp for engaging the spring-loaded button of the handle.

7. The kit of claim 1, in which the removable shaft includes a flat impaction surface to abut the handle, such that a force impacted on the handle transfers to the shaft.

8. A kit for use in implanting a stem into a long bone, the kit including:
a universal handle including a locking mechanism; and
a plurality of shafts, in which each of the plurality of shafts is adapted to couple with the universal handle such that the locking mechanism of the universal handle locks each of the plurality of shafts to the handle.

9. The kit of claim 8, in which the locking mechanism includes a spring-loaded button.

10. The kit of claim 9, in which the handle includes a stop pin adapted to engage a recess in the spring-loaded button.

11. The kit of claim 8, in which each of the plurality of shafts includes an identical end portion to lock with the locking mechanism.

12. The kit of claim 8, in which the plurality of shafts includes at least two of a standard straight shaft, a bullet tip shaft, a curved anterior shaft and a posterior shaft.

13. The kit of claim 8, in which the handle is made of a metal.

14. The kit of claim 8, in which each of the plurality of shafts is made of one of a metal or a disposable plastic.
